# EUROPEAN PATENT APPLICATION

(11) **EP 4 656 632 A1**
(43) Date of publication of application: **03.12.2025**
(21) Application number: 24747481.0
(22) Date of filing: 26.01.2024
(51) Int. Cl.: C07D 307/92, C09K 11/06, C12Q 1/04, G01N 21/64

(54) **PHOSPHOR FOR DETECTING FIRE BLIGHT PATHOGEN AND USE THEREOF**

(30) Priority: 26.01.2023 KR 20230010020
(71) Applicant: UNIVERSITY-INDUSTRY COOPERATION GROUP OF KYUNG HEE UNIVERSITY, Yongin-si, Gyeonggi-do 17104 (KR)
(72) Inventor: KIM, Do-Kyoung, Seoul 02447 (KR); JIN, Ji-Hye, Iksan-si, Jeonbuk-do 54551 (KR)
(74) Representative: Caldon, Giuliano
(86) International application number: PCT/KR2024/001260
(87) International publication number: WO 2024/158248

(57) **Abstract**

The present disclosure relates to a phosphor compound for diagnosing fire blight in fruit trees based on fluorescence and use thereof. The compound is capable of selectively detecting *Erwinia amylovora,* the causal pathogen of fire blight in fruit trees, and emits red fluorescence in response. Due to its rapid diagnostic response and high sensitivity, the compound can be effectively used for diagnosing fire blight in fruit trees through various simple methods such as spraying or swabbing, without the need for specialized equipment.

## Description

### [Technical Field]

The present invention relates to a phosphor for detecting a causal pathogen of fire blight in fruit trees and diagnosing fire blight in fruit trees, and use thereof.

### [Background Art]

Fire blight in fruit trees is a representative plant infectious disease that affects Rosaceae family plants such as pears, apples, and apricots as host plants, and is caused by the pathogen *Erwinia amylovora* (*E. amylovora*)*.* Plants infected with fire blight in fruit trees may exhibit symptoms such as wilting or blackening that resemble burning, which may appear in all plant organs, including blossoms, leaves, branches, and fruits. According to the disease cycle of fire blight in fruit trees, *E. amylovora* may overwinter at low concentrations mainly in branches or infected areas and may spread in the spring via insects, rain, or wind. In particular, when *E*. *amylovora* is exposed to blossoms, one of the major organs of the plant, it may cause infection of the entire host as well as the surrounding soil.

Once fire blight in fruit trees occurs, it is difficult to control and may spread rapidly, making immediate incineration and burial of the infected plants inevitable. In Korea as well, the disease was first reported in 2015 in some orchards in Anseong, Gyeonggi-do, and outbreaks have occurred annually since then. As the affected areas continue to expand, the time and economic costs required to dispose of infected fruit trees are steadily increasing.

Accordingly, so as to control fire blight in fruit trees, it is essential to accurately detect the causal pathogen present in infected plants before the infection spreads. Existing diagnostic methods for fire blight in fruit trees rely on PCR and DNA-based diagnostic strips. Although PCR-based diagnostic methods have high sensitivity, they require skilled personnel and specialized equipment, which limits their field applicability. In addition, DNA-based diagnostic strips involve a somewhat destructive pretreatment process for sample collection, which may cause additional release of pathogens.

Against this background, the inventors of the present disclosure made efforts to develop a field-applicable, non-destructive, and rapid diagnostic method for fire blight in fruit trees, and as a result, developed a phosphor capable of specifically staining *E. amylovora* and confirmed that *E. amylovora* can be detected in actual fruit trees using the phosphor, thus completing the present disclosure.

### [Disclosure]

### [Technical Problem]

Therefore, the present invention has been made in view of the above problems, and it is one object of the present invention to provide a phosphor compound for detecting *Erwinia amylovora* and diagnosing fire blight in fruit trees and a diagnostic method related thereto.

It is another object of the present invention to provide a method of preparing the compound.

It is still another object of the present invention to provide a composition for detecting *Erwinia amylovora* and diagnosing fire blight in fruit trees, including the compound.

It is yet another object of the present invention to provide a method of detecting *Erwinia amylovora* and diagnosing fire blight in fruit trees using the compound.

### [Technical Solution]

In accordance with an aspect of the present invention, the above and other objects can be accomplished by the provision of a compound represented by Formula 1 below:

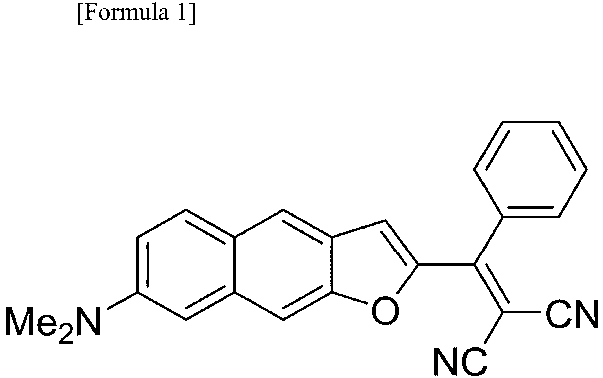

In accordance with another aspect of the present invention, provided is a method of preparing the compound represented by Formula 1, the method including a step of obtaining Compound 3, represented by Formula 3 below, by reacting 6-(dimethylamino)-3-hydroxy-2-naphthaldehyde with bromoacetonitrile and potassium carbonate; a step of obtaining Compound 2, represented by Formula 2 below, by reacting Compound 3 with phenylboronic acid, trifluoroacetic acid, palladium(II) trifluoroacetate and 6,6'-dimethyl-2,2'-dipyridyl; and a step of obtaining the compound represented by Formula 1 by reacting Compound 2 with malononitrile:

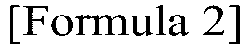

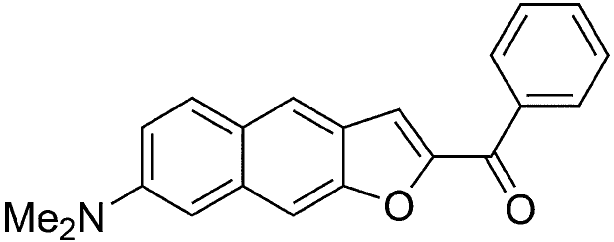

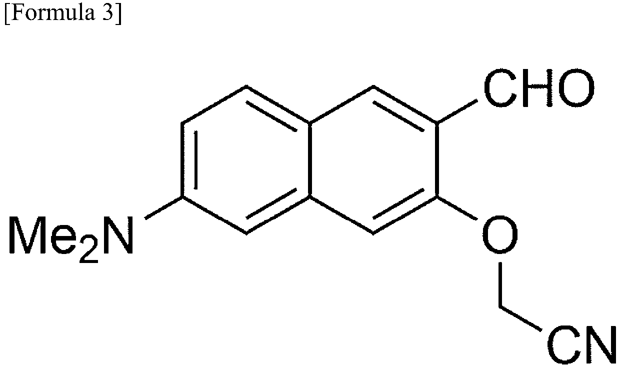

In accordance with still another aspect of the present invention, provided is a composition for detecting *Erwinia amylovora,* including the compound represented by Formula 1 of the present invention.

In accordance with still another aspect of the present invention, provided is a method of detecting *Erwinia amylovora* using the compound represented by Formula 1.

In accordance with still another aspect of the present invention, provided is a composition for diagnosing fire blight in fruit trees, the composition including the compound represented by Formula 1.

In accordance with still another aspect of the present invention, provided is a method of diagnosing fire blight in fruit trees using the compound represented by Formula 1.

In accordance with yet another aspect of the present invention, provided is a composition for screening substances for controlling fire blight in fruit trees and a method of screening using the composition, the composition including the compound represented by Formula 1 and *Erwinia amylovora.*

### [Advantageous effects]

The phosphor compound B-1 synthesized in the present disclosure is capable of detecting *Erwinia amylovora,* the causal pathogen of fire blight in fruit trees, and emits red fluorescence in response. It exhibits high selectivity and sensitivity toward *E. amylovora.*

In addition, the compound can be applied to samples through various simple methods, such as spraying or swabbing, without the need for specialized equipment, and fluorescence can be detected by irradiating with UV-wavelength light through a simple procedure, enabling simple diagnosis of fire blight in fruit trees. Therefore, it can be effectively used for diagnosing fire blight in fruit trees. In particular, compared to conventional methods such as PCR and DNA-based diagnostic strips, it offers faster detection and higher sensitivity, allowing rapid identification of *E. amylovora,* making it highly suitable for use in field environments such as orchards.

### [Description of Drawings]

FIG. 1 is a schematic diagram illustrating the synthetic route of the phosphor (B-1) specific to a causal pathogen (*Erwinia amylovora*) of fire blight in fruit trees of the present invention. Each synthesis step was performed under the following conditions: (a) Bromoacetonitrile, K₂CO₃, DMF, 50°C, 2 h. (b) Phenylboronic acid, Pd(TFA)2, 6,6'-dimethyl-2,2'-dipyridyl, trifluoroacetic acid (TFA), 2-MeTHF, 80°C, 24 h. (c) Malononitrile, pyridine, 70°C, overnight.
FIG. 2 is a graph illustrating the absorption and fluorescence changes of B-1 in response to *E. amylovora.*
FIG. 3 is a graph illustrating the increase in fluorescence intensity of B-1 according to the concentration of *E. amylovora.*
FIG. 4 is a graph illustrating the detection limit of *E. amylovora* using B-1.
FIG. 5 is a graph illustrating the fluorescence change of B-1 over time in response to *E. amylovora.*
FIG. 6 is a set of images illustrating the staining ability of B-1 for *E. amylovora,* in combination with DAPI staining
FIG. 7 illustrates a method and results of detecting *E. amylovora* present on actual plant surfaces using sprayed B-1: (a) A schematic diagram of the method of detecting *E. amylovora* by spraying B-1; (b) Results of detecting various concentrations of *E. amylovora* on immature apples and apple blossoms by spraying B-1; (c, d) Graphs showing the fluorescence intensity from (b); and (e) PCR results for cross-validation of the experiment in (b).
FIG. 8 illustrates a method and results of detecting *E. amylovora* on actual plant surfaces by spraying or swabbing B-1: (a) A schematic diagram illustrating a method of detecting *E*. *amylovora* on actual plant surfaces using spraying or swabbing B-1; (b) Results of detecting *E*. *amylovora* on apple twigs by spraying B-1; (c, d) Results of detecting *E. amylovora* on apple blossoms and leaves using B-1-soaked swabs.
FIG. 9 illustrates a method and results of detecting *E. amylovora* on agricultural tool surfaces by spraying B-1: (a) A schematic diagram illustrating the detection method for *E*. *amylovora* on farming tools by spraying B-1; (b) Results of detecting *E. amylovora* on the surface of pruning shears and a trowel by spraying B-1.

### [Best Mode]

The present inventors sought to develop a novel diagnostic method that overcomes the temporal and spatial limitations of conventional diagnostic methods for fire blight in fruit trees, such as PCR or DNA-based diagnostic strips. They devised a compound capable of specifically reacting with a causal pathogen of fire blight in fruit trees (*Erwinia amylovora*) and, based on the insight that this compound may induce fluorescence enhancement upon reacting with *E*. *amylovora,* developed the phosphor of the present disclosure.

An aspect of the present invention provides a compound represented by Formula 1 below:

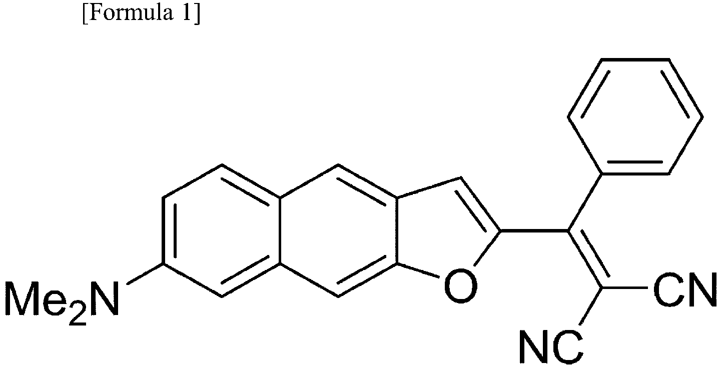

In an embodiment of the present invention, a compound represented by Formula 1 was designated and referred to as 'B-1'.

In an embodiment of the present invention, the compound represented by Formula 1 may be a phosphor capable of detecting the causal pathogen of fire blight in fruit trees. The causal pathogen of fire blight in fruit trees may be *Erwinia amylovora.*

In an embodiment of the present disclosure, the compound represented by Formula 1 may selectively and specifically react with *Erwinia amylovora,* may emit red fluorescence upon reacting with *Erwinia amylovora,* and the intensity of the emitted fluorescence may increase as the concentration of *Erwinia amylovora* increases.

In another embodiment of the present invention, the compound represented by Formula 1 may stain *Erwinia amylovora* present in plants and objects, thereby enabling its visualization.

In an embodiment of the present disclosure, it was confirmed that, when *E. amylovora* was treated with the compound represented by Formula 1 according to the present disclosure, fluorescence significantly increased at 686 nm (Example 2), and that the fluorescence also increased as the concentration of *E. amylovora* increased (Example 3).

In another embodiment of the present disclosure, it was confirmed that the compound represented by Formula 1 is capable of detecting *E. amylovora* at concentrations as low as 10² CFU/mL (Example 4), and exhibited a rapid fluorescence increase within 10 minutes (Example 5).

In another embodiment of the present disclosure, the compound represented by Formula 1 was shown to be capable of staining live *E. amylovora,* and to emit red fluorescence as observed by confocal laser scanning microscopy (Example 6).

In another embodiment of the present disclosure, it was confirmed that the compound represented by Formula 1 is capable of detecting *E. amylovora* on the surfaces of objects and plants (Examples 7 to 9). In Examples 7 and 8, *E. amylovora* at concentrations as low as 10² CFU/mL was effectively detected on apple fruits, branches, and blossoms, and the applicability of methods such as spraying and swabbing was demonstrated. Furthermore, in Example 9, it was confirmed that *E. amylovora* present on the surface of agricultural tools could also be efficiently detected.

Accordingly, the compound represented by Formula 1 may be applied to determine the presence of fire blight in fruit trees by utilizing its specific fluorescence emission in response to *Erwinia amylovora,* the causal pathogen of fire blight in fruit trees.

In an embodiment of the present invention, the compound represented by Formula 1 may be usable for diagnosing fire blight in fruit trees.

In another embodiment of the present invention, the compound represented by Formula 1 may exhibit fluorescence in individuals infected with fire blight in fruit trees.

The individuals infected with fire blight in fruit trees may include plants susceptible to fire blight in fruit trees, soil in which infected plants are cultivated, and tools or equipment that have come into contact with such infected plants or soil. The plant may be a fruit tree and may, for example, include one or more selected from the group consisting of apples, rowan trees, and Rosaceae family plants, but is not limited thereto. The tools or equipment may include agricultural tools and, for example, may include pruning shears, seedling shovels, and the like, but are not limited thereto.

In an embodiment of the present disclosure, the compound represented by Formula 1 may be applied to the surface of an individual infected with fire blight in fruit trees by swabbing, coating, or spraying, and the presence of fire blight in fruit trees may be diagnosed by measuring the fluorescence generated upon irradiation with UV-wavelength light.

In another embodiment of the present disclosure, the UV-wavelength light may be irradiated using a flashlight, lantern, or lamp. The UV-wavelength light may have a wavelength of 350 to 370 nm, preferably 360 to 370 nm, and most preferably 365 nm.

Another aspect of the present invention provides a method of preparing the compound represented by Formula 1, the method including a step of reacting Compound 2 represented by Formula 2 below with malononitrile to obtain the compound represented by Formula 1:

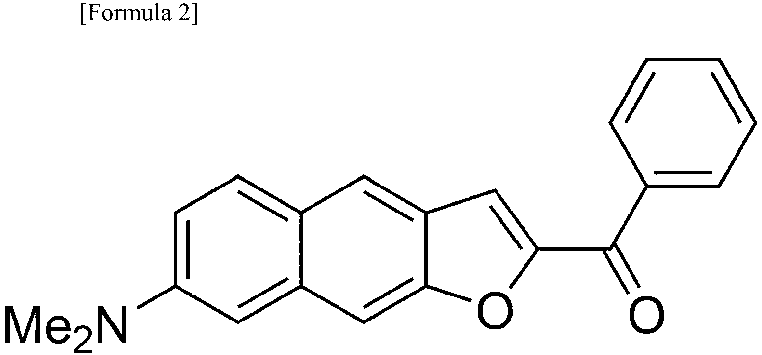

The reaction for obtaining the compound represented by Formula 1 may involve dissolving Compound 2 and malononitrile in pyridine and allowing them to react.

In an embodiment of the present invention, Compound 2 may be obtained by reacting Compound 3 represented by Formula 3 below with phenylboronic acid, trifluoroacetic acid, palladium(II) trifluoroacetate and 6,6'-dimethyl-2,2'-dipyridyl:

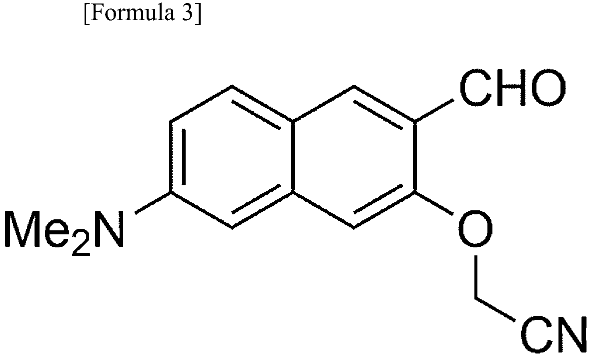

The reaction for obtaining Compound 2 may involve dissolving Compound 3, phenylboronic acid, trifluoroacetic acid, palladium(II) trifluoroacetate, and 6,6'-dimethyl-2,2'-dipyridyl in 2-methyltetrahydrofuran (2-MeTHF) and allowing them to react.

In an embodiment of the present invention, Compound 3 may be obtained by reacting 6-(dimethylamino)-3-hydroxy-2-naphthaldehyde with bromoacetonitrile and potassium carbonate.

The reaction for obtaining Compound 3 may involve mixing 6-(dimethylamino)-3-hydroxy-2-naphthaldehyde, bromoacetonitrile, and potassium carbonate in N, N-dimethyl methanamide (DMF) and allowing them to react.

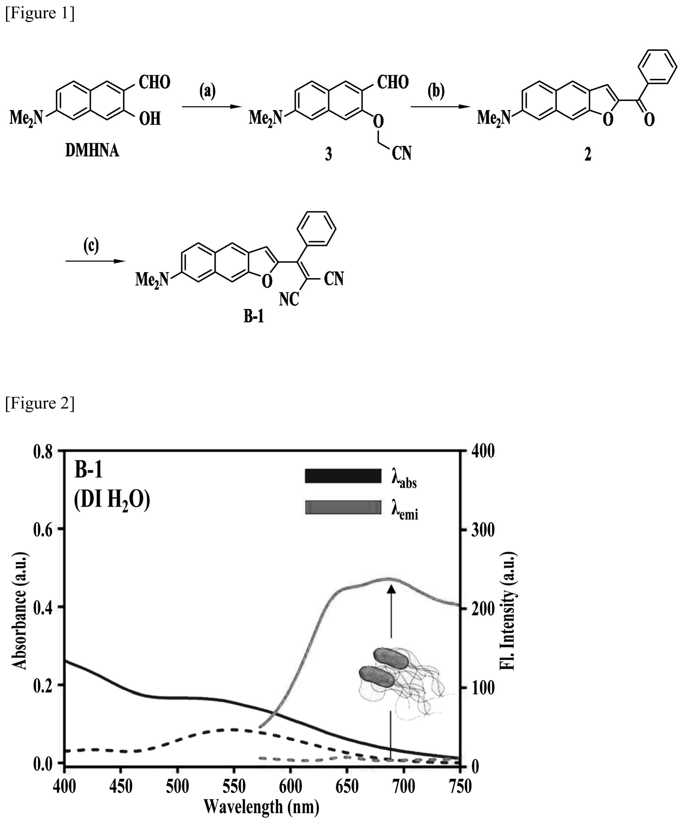

In an embodiment of the present invention, the method of preparing the compound represented by Formula 1 may be represented as in FIG. 1. Preferably, the method may include a step of obtaining Compound 3, represented by Formula 3, by reacting 6-(dimethylamino)-3-hydroxy-2-naphthaldehyde with bromoacetonitrile and potassium carbonate; a step of obtaining Compound 2, represented by Formula 2, by reacting Compound 3 with phenylboronic acid, trifluoroacetic acid, palladium(II) trifluoroacetate and 6,6'-dimethyl-2,2'-dipyridyl; and a step of obtaining the compound represented by Formula 1 by reacting Compound 2 with malononitrile.

Another aspect of the present invention provides a composition for detecting *Erwinia amylovora,* including the compound represented by Formula 1 of the present invention.

In addition, another aspect of the present invention provides a method of detecting *Erwinia amylovora,* the method including a step of adding the compound represented by Formula 1 of the present disclosure to a sample and measuring the resulting fluorescence.

In an embodiment of the present invention, the composition for detecting *Erwinia amylovora* may selectively detect *Erwinia amylovora* in a sample.

In another embodiment of the present invention, the composition for detecting *Erwinia amylovora* may further include one or more selected from the group consisting of a solvent, an acid, a base, and a buffer solution. The composition for detecting *Erwinia amylovora* may be prepared by adding the above-described compound to a solvent, a buffer solution, or a mixture thereof, and then adding an acid and/or a base thereto. In addition, the composition for detecting *Erwinia amylovora* may further include other additives usable in the relevant technical field. The contents of the solvent, acid, base, and buffer solution included in the composition may be appropriately adjusted depending on the required performance. The solvent may include water, THF, methanol, ethanol, an aqueous solution of HI, N, N-dimethylformamide, or a combination thereof.

In an embodiment of the present disclosure, the method of detecting *Erwinia amylovora* may include a step of confirming a change in fluorescence emission or fluorescence intensity of a test sample compared to that of a sample prior to the addition of the compound represented by Formula 1 or a composition for detecting *Erwinia amylovora* including the compound, or compared to that of a normal control sample. Preferably, red fluorescence may be emitted as a result of a selective reaction in the presence of *Erwinia amylovora,* and if the fluorescence emission or fluorescence intensity of the test sample increases compared to that of a sample before the addition of the compound represented by Formula 1 according to the present disclosure, or a composition for detecting *Erwinia amylovora* including the compound, or compared to that of a normal control sample, it may be determined that *Erwinia amylovora* is present.

In another embodiment of the present invention, the step of measuring fluorescence may be performed using any one device selected from the group consisting of a confocal fluorescence microscope, a two-photon fluorescence microscope, an optical coherence tomography scanner, and a two-photon fluorescence microscope and an optical coherence tomography scanner, or may be performed by observing changes in fluorescence emitted upon irradiation with UV-wavelength light from a flashlight, lantern, or lamp applied to a sample to which the compound represented by Formula 1 or a composition for detecting *Erwinia amylovora* including the compound has been added.

In another embodiment of the present invention, the UV-wavelength light may have a wavelength of 350 to 370 nm, preferably 360 to 370 nm, and most preferably, irradiation with light having a wavelength of 365 nm is desirable.

In an embodiment of the present disclosure, the sample may include any of a plant, soil, or object suspected of containing *Erwinia amylovora.* The plant may be a fruit tree and may include one or more selected from the group consisting of pear, apple, rowan tree, and Rosaceae family plants, but is not limited thereto. The object may include tools or equipment such as agricultural tools, and may, for example, include pruning shears, seedling shovels, and the like, but is not limited thereto.

Another aspect of the present invention provides a composition for diagnosing fire blight in fruit trees, including the compound represented by Formula 1 of the present invention.

In addition, another aspect of the present invention provides a method of diagnosing fire blight in fruit trees, the method including a step of adding the compound represented by Formula 1 of the present disclosure to a sample suspected of being infected with fire blight in fruit trees and measuring the resulting fluorescence.

In an embodiment of the present invention, the composition for diagnosing fire blight in fruit trees may diagnose fire blight in fruit trees by selectively detecting *Erwinia amylovora* present in a sample.

In another embodiment of the present invention, the composition for diagnosing fire blight in fruit trees may further include one or more selected from the group consisting of a solvent, an acid, a base, and a buffer solution. The composition for diagnosing fire blight in fruit trees may be prepared by adding the above-described compound to a solvent, a buffer solution, or a mixture thereof, and then adding an acid and/or a base thereto. In addition, the composition for diagnosing fire blight in fruit trees may further include other additives usable in the relevant technical field. The contents of the solvent, acid, base, and buffer solution included in the composition may be appropriately adjusted depending on the required performance. The solvent may include water, THF, methanol, ethanol, aqueous HI, N, N-dimethylformamide, or combinations thereof.

In an embodiment of the present disclosure, the method of diagnosing fire blight in fruit trees may include a step of confirming whether the fluorescence emission or fluorescence intensity of a test sample increases compared to that of a sample prior to the addition of the compound represented by Formula 1 or a composition for diagnosing fire blight in fruit trees including the compound, or compared to that of a normal control sample. Preferably, red fluorescence may be emitted upon infection with fire blight in fruit trees, and if the fluorescence emission or fluorescence intensity of the test sample increases compared to that of a sample before the addition of the compound represented by Formula 1 according to the present disclosure, or a composition for diagnosing fire blight in fruit trees including the same, or compared to that of a normal control sample, the presence of fire blight in fruit trees may be determined.

In an embodiment of the present disclosure, the addition may be performed by applying the compound represented by Formula 1 or a composition for diagnosing fire blight in fruit trees including the same to the surface of a sample suspected of being infected with fire blight in fruit trees by swabbing, coating, or spraying, and the fluorescence may be measured as the fluorescence generated upon irradiation with UV-wavelength light.

In another embodiment of the present disclosure, the step of measuring fluorescence may be performed using any one device selected from the group consisting of a confocal fluorescence microscope, a two-photon fluorescence microscope, and an optical coherence tomography scanner, and may also be performed by observing changes in fluorescence emitted from a sample to which the compound represented by Formula 1 or the composition for diagnosing fire blight in fruit trees including the compound has been added, upon irradiation with UV-wavelength light using a flashlight, lantern, or lamp.

In another embodiment of the present disclosure, the UV-wavelength light may have a wavelength of 350 to 370 nm, preferably 360 to 370 nm, and most preferably, light having a wavelength of 365 nm is irradiated.

In an embodiment of the present disclosure, the sample suspected of being infected with fire blight in fruit trees may include a plant, soil, or object suspected of being infected with fire blight in fruit trees. The plant may be a fruit tree and may include one or more selected from the group consisting of pear, apple, rowan tree, and Rosaceae family plants, but is not limited thereto. The object may include tools or equipment such as agricultural tools and, for example, may include pruning shears, seedling shovels, and the like, but is not limited thereto.

In another embodiment of the present invention, the composition for diagnosing fire blight in fruit trees and the method of diagnosing fire blight in fruit trees may be applied in the field, such as in fruit orchards.

Another aspect of the present invention provides a composition for screening substances for controlling fire blight in fruit trees, including the compound represented by Formula 1 of the present invention and *Erwinia amylovora.*

In addition, another aspect of the present invention provides a method of screening substances for controlling fire blight in fruit trees, the method including a step of adding a candidate substance to *Erwinia amylovora* treated with the compound represented by Formula 1 of the present disclosure and measuring a change in fluorescence.

In an embodiment of the present disclosure, the composition for screening substances for controlling fire blight in fruit trees and the method thereof may involve screening a candidate substance expected to act as a control agent for fire blight in fruit trees by treating the *Erwinia amylovora* (test sample), to which the compound represented by Formula 1 has been applied, with the candidate substance, and determining whether a decrease in the concentration or number of *Erwinia amylovora,* the causal pathogen of fire blight in fruit trees, or inhibition of its increase is observed.

In another embodiment of the present disclosure, the composition for screening substances for controlling fire blight in fruit trees and the method thereof may include a step of adding a candidate substance, which is expected to act as a control agent for fire blight in fruit trees, to a test sample including the compound represented by Formula 1 and *Erwinia amylovora,* and confirming that the fluorescence emission or fluorescence intensity of the test sample decreases compared to that of the sample before addition of the candidate substance. Preferably, in the presence of *Erwinia amylovora,* red fluorescence may be emitted by reaction with the compound represented by Formula 1, and when the fluorescence emission or fluorescence intensity of the test sample to which the candidate substance has been added is decreased compared to that of the test sample before the addition, the candidate substance may be determined to be a substance capable of improving, inhibiting, reducing, or controlling fire blight in fruit trees.

In another embodiment of the present invention, the step of measuring fluorescence may be performed using any one device selected from the group consisting of a confocal fluorescence microscope, a two-photon fluorescence microscope, an optical coherence tomography scanner, and a two-photon fluorescence microscope and an optical coherence tomography scanner, and may also be performed by observing changes in fluorescence emitted from the test sample upon irradiation with UV-wavelength light using a flashlight, a lantern, or a lamp.

In another embodiment of the present disclosure, the UV-wavelength light may have a wavelength of 350 to 370 nm, preferably 360 to 370 nm, and most preferably, irradiation with light having a wavelength of 365 nm is desirable.

Hereinafter, preferred embodiments are provided to aid in the understanding of the present disclosure. However, the following embodiments are merely illustrative for easier understanding of the present disclosure, and the scope of the present disclosure is not limited thereto.

### Example 1. Synthesis and purification of E. amylovora-specific phosphor (B-1)

The present inventors performed organic synthesis as shown in FIG. 1, based on the compound disclosed in Korean Patent No. 10-1524915, to develop *E. amylovora*-specific phosphor (B-1).

### 1-1. Synthesis of Compound 3

Specifically, 6-(dimethylamino)-3-hydroxy-2-naphthaldehyde (DMHNA, 30 mg, 0.139 mmol), bromoacetonitrile (14 µL, 0.209 mmol), and potassium carbonate (29 mg, 0.209 mmol) as starting materials for the synthesis were added to a flame-dried 5 mL round-bottom flask containing 1.5 mL of N, N-dimethyl methanamide (DMF). An argon balloon was attached, and the mixture was stirred at 50 °C for 2 hours using a silicone oil bath. The reaction mixture was then extracted with ethyl acetate (EtOAc, 100 mL), deionized water (DI H₂O, 100 mL), and saturated brine (30 mL). The collected organic layer was dried over anhydrous sodium sulfate (Na₂SO₄, 5 g), and the solvent was removed using a rotary evaporator. The crude product was purified by silica gel column chromatography using 30% EtOAc/hexane (v/v) as an eluent, yielding Compound 3 as a yellow solid (yield: 92%).

¹H NMR (500 MHz, CDCl₃): 3.13 (s, 6H), 4.97 (s, 2H), 6.77 (d, 1H), 7.04 (s, 1H), 7.05-7.07 (m, 1H), 7.74-7.78 (d, 1H), 8.25 (s, 1H), 10.34 (s, 1H).

¹³C NMR (125 MHz, CDCl₃): 40.5, 53.8, 104.4, 106.0, 115.0, 115.3, 121.6, 121.8, 131.4, 132.9, 139.3, 151.1, 155.1, 188.6.

HRMS (ESI) m/z: [M + H] ⁺calcd for C ₁₅H ₁₄N ₂O ₂, 254.1055; found, 254.1057

### 1-2. Synthesis of Compound 2

Specifically, Compound 3 (29 mg, 0.115 mmol), phenylboronic acid (28 mg, 0.230 mmol), trifluoroacetic acid (88 µL, 1.15 mmol), palladium(II) trifluoroacetate (30 mg, 0.09 mmol), and 6,6'-dimethyl-2,2'-dipyridyl (33 mg, 0.180 mmol) as starting materials for the synthesis were dissolved in 2-methyltetrahydrofuran (1.5 mL), and the resulting solution was transferred to a flame-dried Schlenk tube and stirred at room temperature for 15 minutes. The reaction mixture was then heated at 80°C for an additional 24 hours using a silicone oil bath. After the reaction, the product was extracted with ethyl acetate (EtOAc, 100 mL), deionized water (DI H₂O, 100 mL), and saturated brine (30 mL). The collected organic layer was dried over anhydrous sodium sulfate (Na₂SO₄, 5 g), and the solvent was removed using a rotary evaporator. The crude product was then purified by silica gel column chromatography using 10% EtOAc/hexane (v/v) as an eluent, affording Compound 2 as an orange solid (yield: 41%).

¹H NMR (500 MHz, (CD₃)₂SO, δ): 3.06 (s, 6H), 7.02-7.03 (d, 1H), 7.25-7.28 (m, 1H), 7.61-7.64 (m, 2H), 7.71-7.75 (m, 1H), 7.83 (d, 1H), 7.89-7.92 (m, 2H), 7.99-8.01 (m, 2H), 8.23 (s, 1H).

¹³C NMR (125 MHz, (CD₃)₂SO, δ): 40.8, 105.1, 105.4, 116.1, 117.7, 122.3, 124.4, 125.1, 128.7, 129.5, 129.6, 132.8, 136.1, 137.8, 148,8, 152.6, 155.4, 184.7.

HRMS (ESI) m/z: [M + H] ⁺calcd for C ₂₁H₁₇NO₂, 315.1259; found, 315.1261

### 1-3. Synthesis of Compound B-1

Specifically, Compound 2 (10 mg, 0.032 mmol), malononitrile (4 mg, 0.063 mmol), and pyridine (1.5 mL) as starting materials for the synthesis were added to a flame-dried round-bottom flask. A reflux condenser was connected, an argon balloon was attached, and the mixture was stirred overnight at 70 °C. After the reaction, the solvent was removed using a rotary evaporator, and the product was extracted with EtOAc, deionized water (DI H₂O), and saturated brine. The collected organic layer was dried over anhydrous sodium sulfate (Na₂SO₄, 5 g), and the solvent was removed using a rotary evaporator. The crude product was purified by silica gel column chromatography using 20% EtOAc/hexane (v/v) as an eluent, yielding compound B-1 as a purple solid (yield: 41%).

¹H NMR (500 MHz, CD₃COCD₃, δ): 3.14 (s, 6H), 7.10 (d, 1H), 7.21 (d, 1H), 7.27-7.30 (m, 1H), 7.64 (t, 1H), 7.66 (t, 1H), 7.68-7.70 (m, 1H), 7.71-7.73 (m, 2H), 7.79 (s, 1H), 7.79 (s, 1H), 7.87 (d, 1H), 8.14 (s. 1H).

¹³C NMR (500 MHz, CD₃COCD₃): 40.5, 78.8, 105.0, 105.3, 114.9, 115.3, 117.2, 122.7, 123.6, 125.3, 126.1, 129.7, 130.5, 130.8, 132.0, 132.2, 134.8, 138.0, 150.4, 152.3, 156.4, 157.4.

HRMS (ESI) m/z: [M + H] ⁺calcd for C ₂₄H₁₇N₃O, 363.1372; found, 363.1369

### Example 2. Evaluation of absorption and fluorescence properties of B-1

To evaluate the absorption and fluorescence properties of the phosphor compound B-1 synthesized in Example 1, the changes in the absorption and fluorescence spectra of B-1 were measured in aqueous solution and in the presence of *Erwinia amylovora,* the causal pathogen of fire blight in fruit trees, and the results are shown in FIG. 2.

Specifically, the *E. amylovora* used in the experiment was cultured overnight at 26 °C in Luria-Bertani (LB) liquid medium. After cultivation, *E. amylovora* was harvested by centrifugation at 10,000 rcf. First, B-1 was added, and its absorption and fluorescence spectra were analyzed in aqueous solution. Then, *E. amylovora* (4.3 × 10⁸ CFU) was added to the aqueous solution containing B-1, and the changes in the absorption and fluorescence spectra were analyzed. A UV/Vis spectrophotometer (Agilent, USA) was used to analyze the absorbance spectra, and a fluorescence spectrophotometer (SHIMADZU CORP. RF-6000, Japan) was used for fluorescence spectra analysis. For all spectral measurements, a standard quartz cell with a path length of 1 cm (Hellma Analytics, Germany) was used.

The measurement results are shown in FIG. 2, where the absorption and fluorescence spectra of B-1 (10 µM) in aqueous solution are represented by a dashed line, and those in the presence of *E. amylovora* are represented by a solid line.

In aqueous solution, B-1 exhibited maximum absorption at 550 nm, and when fluorescence was measured using this wavelength as the excitation wavelength, little to no fluorescence was observed. Upon addition of *E. amylovora* (4.3 × 10⁸ CFU), the absorption spectrum showed a slight overall increase, and fluorescence measured at 550 nm excitation exhibited a significant enhancement compared to the aqueous condition alone. The highest fluorescence increase was observed at 686 nm, which lies in the near-infrared region. These results confirm that B-1 detects *E. amylovora* and emits red fluorescence in response.

### Example 3. Fluorescence response of B-1 according to concentration of E. amylovora

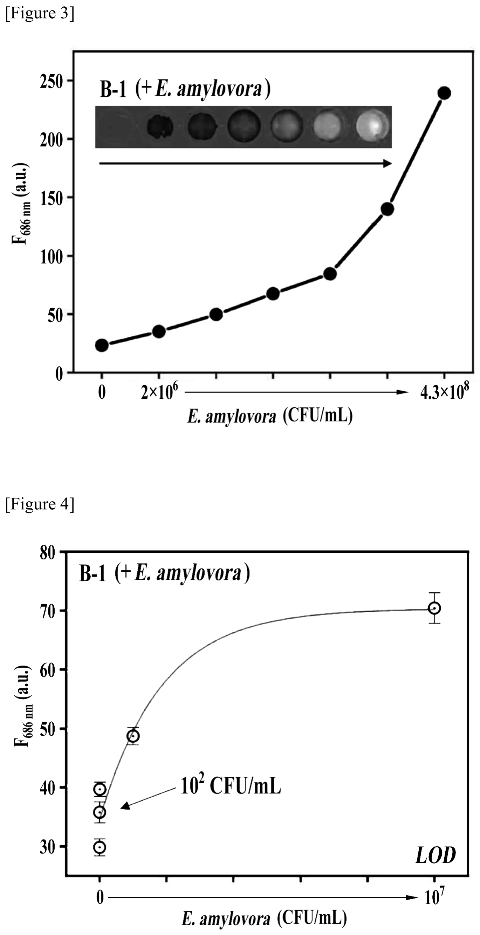

The fluorescence response of the phosphor compound B-1 prepared in Example 1 was measured at varying concentrations of *E. amylovora,* and the results are presented in FIG. 3.

Specifically, to determine the concentration of *E. amylovora* obtained as described in Example 2, absorbance at 600 nm was measured (OD₆₀₀ 0.1 = 1 × 10⁸ cells). *E. amylovora was* then diluted in DI H₂O to concentrations ranging from 2 × 10⁶ to 4 × 10⁸ CFU/mL, and each dilution was reacted with 10 µM of B-1. Fluorescence intensity at 686 nm was recorded after excitation at 550 nm, and plotted on a graph. As a result, the fluorescence of B-1 increased in proportion to the concentration of *E. amylovora,* indicating a positive correlation. The inset image in the graph shows the reaction of *E. amylovora* and B-1 under the same concentration conditions in a 96-well plate, captured using a fluorescence imaging system (VISQUE In Vivo Smart-LF, Korea). Consistent with the graph result, an increase in fluorescence was observed with increasing concentrations of *E. amylovora.*

### Example 4. Determination of limit of detection of B-1 for E. amylovora

*E. amylovora* was serially diluted and reacted with the phosphor compound B-1 synthesized in Example 1 to evaluate its minimum detection capability, and the results are shown in FIG. 4.

Specifically, quantified *E. amylovora,* as prepared in Example 3, was reacted with 5 µM of B-1 in DI H₂O. Fluorescence intensity was then measured at 686 nm with an excitation wavelength of 551 nm, and the results were plotted on a graph.

As a result of the reaction with serially diluted *E. amylovora,* it was confirmed that detection was possible at concentrations as low as 10² CFU/mL. This concentration is close to that of *E. amylovora* overwintering during the winter season, and the method demonstrated significantly high sensitivity even when compared with conventional diagnostic methods such as PCR and DNA-based diagnostic strips.

### Example 5. Time-dependent detection capability of B-1 for E. amylovora

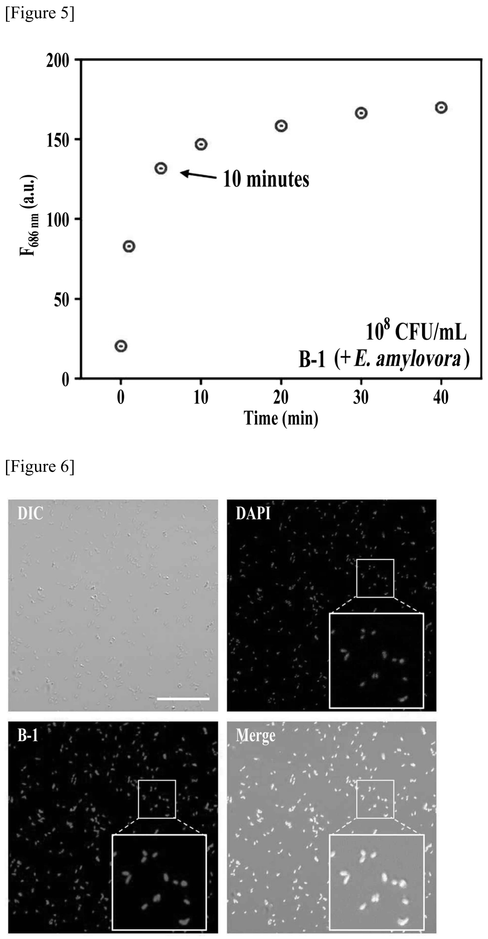

The time-dependent fluorescence response of the phosphor compound B-1 synthesized in Example 1 was evaluated in the presence of *E. amylovora,* and the results are shown in FIG. 5.

Specifically, 10 µM of B-1 was reacted with *E. amylovora* at a concentration of 10⁸ CFU/mL, quantified as described in Example 3, in DI H₂O. Fluorescence changes were monitored at 686 nm with 550 nm excitation for up to 40 minutes. As shown in FIG. 5, B-1 exhibited a rapid fluorescence increase within 10 minutes upon reaction with *E. amylovora.* These results confirm that B-1 possesses fast detection capability for *E. amylovora.*

### Example 6. Evaluation of staining ability of B-1 for E. amylovora

The staining ability of the phosphor compound B-1 synthesized in Example 1 for *E*. *amylovora* was evaluated, and the results are shown in FIG. 6.

Specifically, *E. amylovora* obtained as described in Example 2 was stained by reacting with 30 µM of B-1 in phosphate-buffered saline (PBS) for 1 hour. To visualize only live *E*. *amylovora,* a counterstain with 300 nM of 4',6-diamidino-2-phenylindole (DAPI) was applied simultaneously. The stained *E. amylovora* was then washed three times with PBS to remove residual B-1 and DAPI, followed by heat fixation and mounting on a slide glass for imaging using a confocal laser scanning microscope (Carl Zeiss, Germany). During confocal microscopy, differential interference contrast (DIC) images were also captured. The wavelengths used for imaging were as follows: [DAPI] excitation wavelength: 405 nm, emission wavelength: 410-530 nm, [B-1] excitation wavelength: 561 nm, emission wavelength: 600-700 nm.

FIG. 6 illustrates the results of confocal laser scanning microscopy, presenting images obtained for DIC, DAPI, B-1, and a merged image of all channels. As a result, both DAPI and B-1 fluorescence signals were observed in *E. amylovora* identified in the DIC image. The complete overlap of the fluorescence signals confirmed that B-1 is capable of staining live *E*. *amylovora* and produces strong red fluorescence.

### Example 7. Evaluation of B-1's responsiveness to E. amylovora on plant surfaces (spraying method)

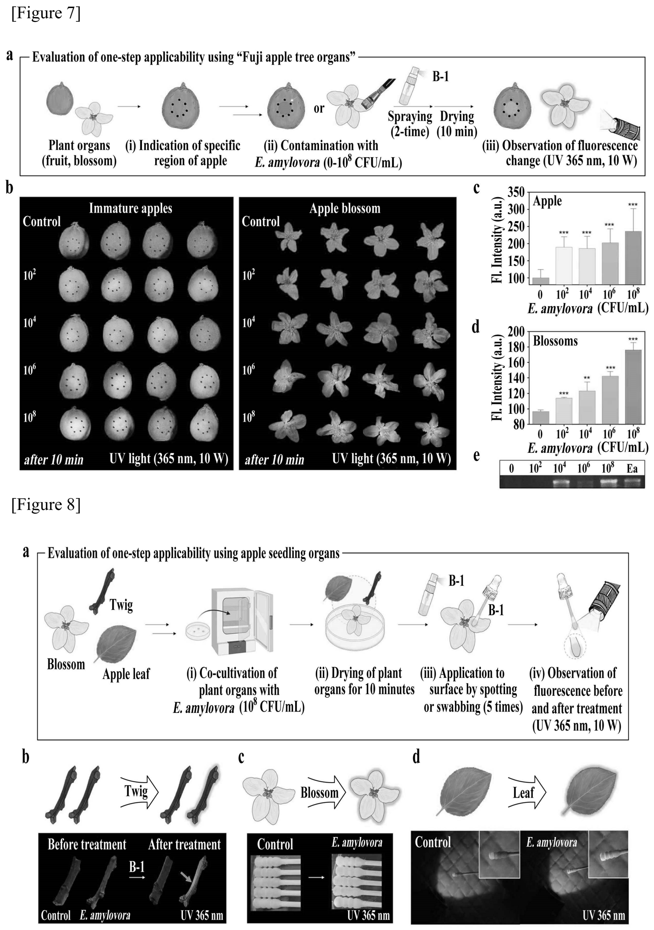

To evaluate whether *E. amylovora* present on actual plant surfaces could be detected using the phosphor compound B-1 synthesized in Example 1, a spraying-based detection was performed by applying B-1 to tissues (organs: fruits and blossoms) of a Fuji apple tree, and the procedure and results are shown in FIG. 7.

Specifically, to simulate plants infected with fire blight in fruit trees, *E. amylovora* quantified as described in Example 3 at concentrations of 10² to 10⁸ CFU/mL was applied to immature apples (fruits) and apple blossoms using a brush, as shown in FIG. 7a. To allow *E*. *amylovora* to adhere to the plant surface, the samples were air-dried for 10 minutes. For detection, a 100 µM solution of B-1 diluted in DI H₂O was sprayed twice, and 10 minutes later, fluorescence changes were observed under a 365 nm flashlight.

As a result, red fluorescence was observed for all concentrations of *E. amylovora* applied to both immature apples and apple blossoms (FIG. 7b). Using the ImageJ software, the fluorescence intensity of B-1 was quantified in each region of interest according to the applied concentration of *E. amylovora,* and the results were plotted in FIGS. 7c and 7d. The analysis showed a significant increase in fluorescence intensity with increasing *E. amylovora* concentration, confirming that detection was possible on actual plant surfaces at levels as low as 10² CFU/mL.

In addition, to cross-validate the detection of *E. amylovora* based on the fluorescence response of B-1, PCR analysis, a conventional method for diagnosing fire blight in fruit trees, was performed, and the results are shown in FIG. 7e. The PCR targeted the pEA29 plasmid, a known DNA marker of *E. amylovora,* and templates included petals from FIG. 7a that showed fluorescence after B-1 spraying, as well as *E. amylovora* (positive control). A single fluorescent petal was placed in a 1.75 mL tube containing 100 µL of DI H₂O and crushed more than 10 times using a pipette tip. The primers used were as follows: Forward: GCACTGAGGTTTTTAGGGATATCCGCTG (SEQ ID NO: 1), Reverse: GCTCAATCGCCAGGGAAATGATATCGGC (SEQ ID NO: 2). PCR amplification was performed using Platinum^{™} II Taq Hot-Start DNA Polymerase (Invitrogen) according to the manufacturer's instructions.

As a result, the PCR findings closely matched the detection results obtained through B-1 spraying, confirming that B-1 is capable of detecting *E. amylovora* present on plant surfaces with high sensitivity.

### Example 8. Evaluation of B-1's responsiveness to E. amylovora on plant surfaces (spray and swab methods)

The phosphor compound B-1 synthesized in Example 1 was applied using various methods to detect *E. amylovora* on plant surfaces, and the results are shown in FIG. 8.

Specifically, as shown in FIG. 8a, apple tree tissues (twig, blossom, and leaf) were incubated overnight at 26 °C on LB agar plates along with *E. amylovora* (10⁸ CFU/mL) to simulate infection with fire blight in fruit trees. Detection of *E. amylovora* on each plant tissue was then attempted by either spraying with a 100 µM solution of B-1 or swabbing with a cotton swab soaked in B-1 solution. Fluorescence changes were observed under a 365 nm flashlight.

As a result, red fluorescence was observed when B-1 was sprayed onto apple twigs infected with fire blight in fruit trees (FIG. 8b). Similarly, when a cotton swab soaked with B-1 was rubbed onto infected apple blossoms and leaves, red fluorescence was emitted from the swab (FIGS. 8c and 8d). These findings confirm that B-1 can be applied through various methods for the detection of *E. amylovora* on plant surfaces.

### Example 9. Evaluation of B-1's responsiveness to E. amylovora on agricultural tool surfaces

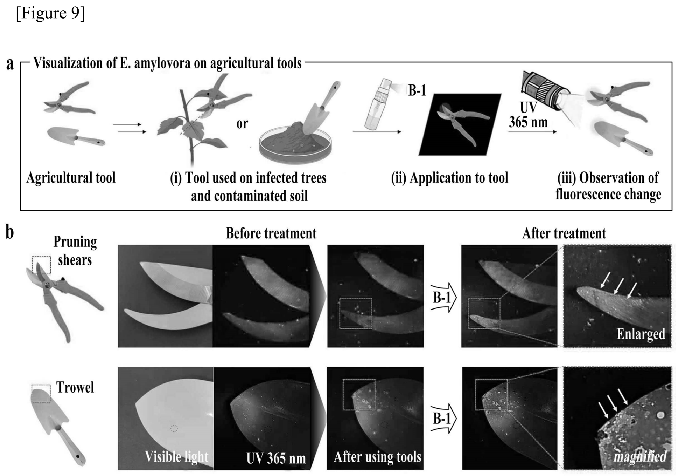

To evaluate whether the phosphor compound B-1 synthesized in Example 1 could detect *E. amylovora* present on objects, a spraying-based detection method was employed. As the test object, farming tools, which are known to be one of the causes of cross-contamination from infected plants in orchards, were used. The procedure and results are shown in FIG. 9.

Specifically, to simulate farming tools exposed to *E. amylovora,* pruning shears and a trowel were either used to cut fire blight-infected plants directly or rubbed in soil contaminated with *E. amylovora,* as shown in FIG. 9a. Detection of *E. amylovora* was carried out by spraying 100 µM of B-1 solution twice onto the tool surfaces, and fluorescence images were captured under a 365 nm flashlight.

As shown in FIG. 9b, the areas of the pruning shears and trowel contaminated with *E*. *amylovora* emitted red fluorescence, confirming that B-1 can be used to detect *E. amylovora* on object surfaces.

The foregoing description of the present disclosure is intended for illustrative purposes only, and it will be understood by those skilled in the art to which the present disclosure pertains that various modifications and other equivalent forms may be made without departing from the spirit or essential characteristics of the present disclosure. Therefore, the above-described embodiments should be understood as illustrative in all respects and not as limiting.

## Claims

1. A compound represented by Formula 1 below:

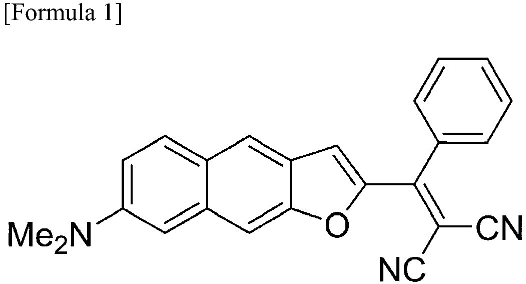

2. The compound according to claim 1, wherein the compound is a phosphor capable of detecting a causal pathogen of fire blight in fruit trees.

3. The compound according to claim 2, wherein the compound emits red fluorescence in response to *Erwinia amylovora.*

4. The compound according to claim 2, wherein fluorescence emitted by the compound increases as a concentration of *Erwinia amylovora* increases.

5. The compound according to claim 1, wherein the compound is usable for diagnosing fire blight in fruit trees.

6. The compound according to claim 5, wherein the compound exhibits fluorescence in an individual infected with fire blight in fruit trees.

7. The compound according to claim 5, wherein the compound is applied to a surface of an individual infected with fire blight in fruit trees by swabbing, coating, or spraying, and fluorescence generated upon irradiation with UV-wavelength light is measured to diagnose infection with fire blight in fruit trees.

8. A method of preparing the compound according to claim 1, the method comprising: reacting Compound 2 represented by Formula 2 below with malononitrile to obtain the compound represented by Formula 1 according to claim 1:

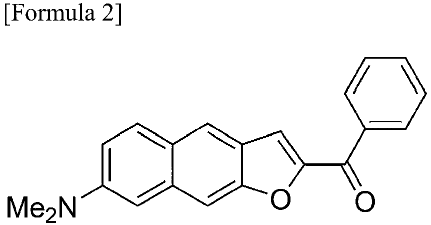

9. The method according to claim 8, wherein Compound 2 is obtained by reacting Compound 3 represented by Formula 3 below with phenylboronic acid, trifluoroacetic acid, palladium(II) trifluoroacetate and 6,6'-dimethyl-2,2'-dipyridyl:

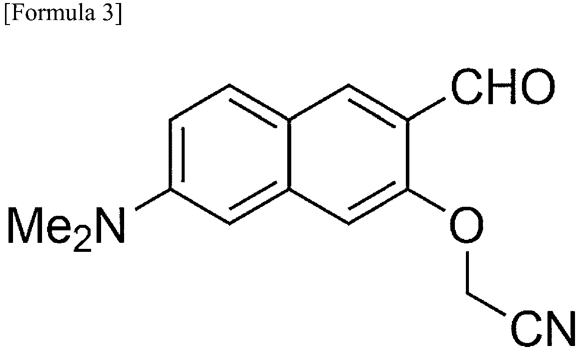

10. The method according to claim 9, wherein Compound 3 is obtained by reacting 6-(dimethylamino)-3-hydroxy-2-naphthaldehyde with bromoacetonitrile and potassium carbonate.

11. A composition for detecting *Erwinia amylovora,* the composition comprising the compound according to claim 1.

12. A method of detecting *Erwinia amylovora,* the method comprising measuring fluorescence generated by adding the compound according to claim 1 to a sample.

13. A composition for diagnosing fire blight in fruit trees, the composition comprising the compound according to claim 1.

14. A method of diagnosing fire blight in fruit trees, the method comprising measuring fluorescence generated by adding the compound according to claim 1 to a sample suspected of being infected with fire blight in fruit trees.

15. A composition for screening substances for controlling fire blight in fruit trees, the composition comprising the compound according to claim 1 and *Erwinia amylovora.*
